# EUROPÄISCHE PATENTSCHRIFT

(11) **EP 2 349 092 B1**
(45) Veröffentlichungstag und Bekanntmachung des Hinweises auf die Patenterteilung: **04.01.2017**
(21) Anmeldenummer: 09777764.3
(22) Anmeldetag: 08.08.2009
(51) Int. Cl.: A61F 2/16

(54) **VORRICHTUNG ZUM IMPLANTIEREN EINER INTRAOKULARLINSE IN EIN AUGE**
DEVICE FOR IMPLANTING AN INTRAOCULAR LENS INTO AN EYE
DISPOSITIF PERMETTANT D'IMPLANTER UNE LENTILLE INTRA-OCULAIRE DANS UN OEIL

(30) Priorität: 14.08.2008 DE 102008037697
(43) Veröffentlichungstag der Anmeldung: 03.08.2011
(73) Patentinhaber: Carl Zeiss Meditec AG, 07745 Jena (DE)
(72) Erfinder: PANKIN, Dmitry, 13355 Berlin (DE)
(74) Vertreter: Böhmer, Sabine
(86) Internationale Anmeldenummer: PCT/EP2009/005772
(87) Internationale Veröffentlichungsnummer: WO 2010/017933

(56) Entgegenhaltungen:
- WO-A1-2007/005692
- US-A- 6 162 229

## Beschreibung

Die Erfindung betrifft eine Vorrichtung zum Implantieren einer Intraokularlinse in ein Auge mit einer Injektionskartusche und mit einem durch ein Kartuschenlumen schiebbaren Stößel, mit welchem die Intraokularlinse durch ein sich verjüngendes distales Kartuschenende geschoben werden kann, wobei an einem distalen Ende des Stößels ein Stempelabschnitt vorgesehen ist, der unter Dichtwirkung an der Innenwand des Kartuschenlumens anliegt und mit einem Frontbereich auf die Intraokularlinse wirkt.

Eine Vorrichtung der eingangs genannten Art ist aus der EP 1 438 929 B1 bekannt. Dort ist eine Injektionskartusche für eine Intraokularlinse beschrieben. Die Injektionskartusche hat ein Kartuschenlumen, in dem mittels eines Stößels eine gefaltete oder gerollte Intraokularlinse bewegt werden kann. Die Injektionskartusche hat in einem Frontabschnitt ein distales Kartuschenende, in dem eine Austrittsöffnung vorgesehen ist. Der Querschnitt des Frontabschnitts ist mit einer Verjüngung ausgebildet. Für das Implantieren einer Intraokularlinse in einem Patientenauge wird mittels des Stößels die im Kartuschenlumen angeordnete Intraokularlinse in der Injektionskartusche zu dessen Austrittsöffnung und dann durch die Austrittsöffnung bewegt. Der Stößel hat ein distales Stößelende, das auf die Intraokularlinse wirkt. Dieses Stößelende weist eine gummielastische Dichtung mit einer umlaufenden Dichtlippe auf, welche an der Innenwand des Kartuschenlumens anliegt.

Injektionskartuschen für Intraokularlinsen, die einen Frontabschnitt mit geringem Querschnitt haben, ermöglichen es, eine Intraokularlinse durch eine entsprechend kleine Inzision in ein Patientenauge einzubringen.

Je geringer der Querschnitt des Frontabschnitts einer Intraokularlinse ist, desto größere Kräfte sind erforderlich, um die Intraokularlinse durch diese hindurch über die Öffnung aus der Injektionskartusche herauszubewegen. Damit beim Verschieben der Intraokularlinse durch den sich verjüngenden Bereich der Injektionskartusche diese keinen Schaden nimmt, ist eine möglichst große Angriffsfläche erforderlich, mit welcher der Stößel auf die Intraokularlinse wirkt.

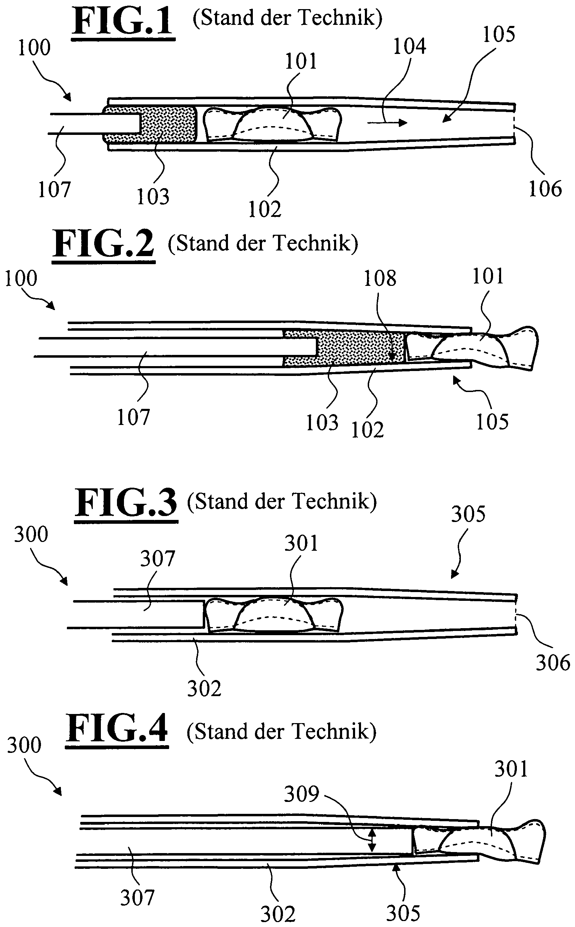

Aus der WO 2007/005692 A1 ist eine Vorrichtung zum Implantieren einer Intraokularlinse bekannt, deren Bauform in Fig. 1 und Fig. 2 gezeigt ist und die nachfolgend erläutert wird: Die Vorrichtung 100 zum Implantieren einer Intraokularlinse 101 umfasst eine Injektionskartusche 102, in der sich zum Zwecke der Injektion in das Auge eines Patienten eine Intraokularlinse 101 befindet. Die Intraokularlinse 101 kann in der Injektionskartusche 102 in Richtung des Pfeils 104 durch einen sich verjüngenden Frontabschnitt 105 zu einer Austrittsöffnung 106 verschoben werden. Hierzu ist ein Stößel 107 vorgesehen, der von einem Operateur bewegt werden kann. Dem Stößel 107 ist ein Stempelabschnitt 103 zugeordnet. Der Stößel 107 wirkt mittels des Stempelabschnitts 103 auf die Intraokularlinse 101. Silikon ist ein sehr elastisches Material und lässt sich entsprechend leicht verformen. Der Stößel 107 besteht aus festem Material. Demgegenüber ist der Stempelabschnitt 103 mit einem Körper aus verformbarem Silikon ausgeführt.

Wenn, wie in Fig. 2 gezeigt, die Intraokularlinse 101 mittels des Stößels 107 durch den sich verjüngenden Frontabschnitt 105 der Vorrichtung 100 zum Implantieren einer Intraokularlinse bewegt wird, ist eine starke Verformung des Stempelabschnitts 103 in Form des Körpers aus Silikon die Folge. Dies bewirkt dort große Druckkräfte auf die Innenwandung 108 der Injektionskartusche 102. Es besteht dann die Gefahr, dass bei Injizieren der Intraokularlinse 101 in ein Patientenauge der Frontabschnitt 105 der Injektionskartusche 102 platzt.

In der FR 2 900 570 A1 ist eine Vorrichtung zum Implantieren einer Intraokularlinse mit einer Injektionskartusche beschrieben, die in Fig. 3 gezeigt ist. Die Vorrichtung 300 ist aus Kunststoffmaterial. Um die Intraokularlinse 301 in der Injektionskartusche 302 aus einer Parkposition durch einen mit Verjüngung ausgeführten Frontabschnitt 305 mit Austrittsöffnung 306 zu bewegen, ist ein Stößel 307 aus Metall oder festem Kunststoff vorgesehen.

Die Fig. 4 zeigt die Vorrichtung 300 zum implantieren einer Intraokularlinse 301 in einer Einstellung, in der die Intraokularlinse 301 durch den sich verjüngenden Frontabschnitt der Injektionskartusche 302 bewegt wird. Da der Stößel aus festem Material besteht, legt dessen Durchmesser 309 den Querschnitt des Frontabschnitts 305 der Injektionskartusche 302 fest. Bei Einsatz eines Stößels aus festem Material ergibt sich eine vergleichsweise große Öffnung 306 der Injektionskartusche 302.

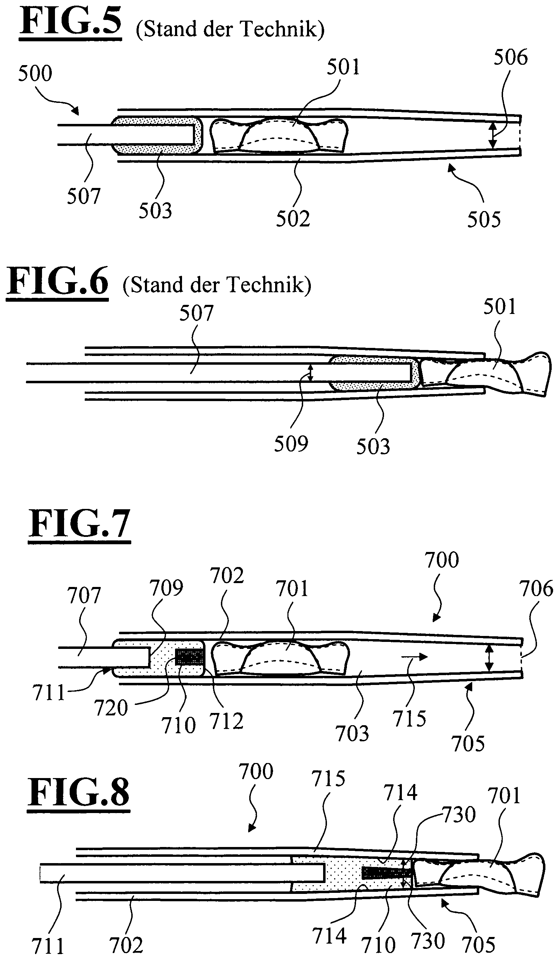

In Fig. 5 ist eine aus dem Stand der Technik bekannte Vorrichtung 500 zum Implantieren einer Intraokularlinse 501 in Auge gezeigt, die einen Stößel 507 aus festem Material enthält, dem ein Stempelabschnitt 503 aus komprimierbarem Schaumstoff in Form von Hartschaum zugeordnet ist. Dies ermöglicht es, den Frontabschnitt 505 der Injektionskartusche 502 mit einem Querschnittsdurchmesser 506 auszubilden, der geringer ist als bei der aus der FR 2 900 570 A1 bekannten Bauform.

Die Fig. 6 zeigt eine Einstellung der Vorrichtung 500 zum Implantieren einer Intraokularlinse 501 in Auge, bei der mittels des Stößels 507 die Intraokularlinse 501 in den Frontabschnitt der Vorrichtung 500 bewegt ist.

Hartschaum ist sehr gut komprimierbar und lässt sich in allen Richtungen sehr leicht verformen. Um ein Verkeilen des Körpers 503 aus Hartschaum in der Injektionskartusche 502 zu vermeiden, ist eine Stabilisierung des Körpers 503 durch den Stößel 507 erforderlich: Der Stößel 507 muss weit in den Körper 503 aus Hartschaum hineinragen.

Wie die Fig. 6 zeigt, hat das zur Folge, dass der Durchmesser 506 des Querschnitts am Frontabschnitt 505 der Injektionskartusche 502 wiederum dem Durchmesser 509 des Stößels 503 entsprechen muss.

US 6,162,229 und WO 2007/005692 A1 zeigen jeweils eine Vorrichtung zum Implantieren einer Intraokularlinse, wobei sich ein Stößel in ein sich verjüngendes distales Ende einer Injektionskartusche schieben lässt, und wobei am distalen Ende des Stößels ein Stempelabschnitt mit einem Hohlraum vorgesehen ist.

Aufgabe der Erfindung ist es, eine Vorrichtung zum Implantieren einer Intraokularlinse in ein Patientenauge mit einer Injektionskartusche bereitzustellen, welche mit einem sich verjüngenden Frontabschnitt ausgeführt werden kann, dessen Durchmesser gering ist, der eine sehr kleine Austrittsöffnung hat, und bei der beim Injizieren der Intraokularlinse in ein Patientenauge keine großen Druckkräfte auf die Innenwandung der Injektionskartusche im Frontabschnitt auftreten und auch keine großen Reibungskräfte auftreten

Diese Aufgabe wird durch eine Vorrichtung zum Implantieren einer Intraokularlinse gemäß Anspruch 1 gelöst, bei welcher in dem Stempelabschnitt ein Hohlraum mit einer derartigen Hohlraumgeometrie ausgebildet ist, dass Druckkräfte des Stempelabschnitts auf die Innenwand des Kartuschenlumens im Bereich des sich verjüngenden Kartuschenendes minimiert werden.

Diese Bauform für eine Vorrichtung zum Implantieren einer Intraokularlinse ermöglicht gegenüber der aus der EP 1 438 929 B1 bekannten Bauform, in einer Injektionskartusche eine Intraokularlinse mit einem Stempelabschnitt zu bewegen, der eine vergleichsweise große Stirnfläche aufweist.

Gemäß der Erfindung enthält der Hohlraum ein Spannelement, das bei einer Deformation des Hohlraums eine auf die Hohlraumwand gerichtete Kraft ausübt. Auf diese Weise wird ein sicheres Anliegen der Oberfläche des Stempelabschnitt an der Innenwandung der Injektionskartusche gewährleistet.

In Weiterbildung der Erfindung hat der Stempelabschnitt eine Symmetrieachse bezüglich der die auf die Hohlraumwand gerichtete Kraft bei Deformation des Stempelabschnitts in dem sich verjüngenden distalen Kartuschenende radialsymmetrisch ist. Auf diese Weisen können unerwünschte Kraftspitzen beim Bewegen des Stempelabschnitts in der Injektionskartusche vermieden werden.

In Weiterbildung der Erfindung ist das Spannelement eine Materialfüllung des Hohlraums, vorzugsweise Hartschaum. Auf diese Weise kann ein sehr kostengünstiges Spannelement für den Stempelabschnitt bereitgestellt werden. Es ist jedoch auch möglich, als Spannelement eine Hülse mit wenigstens einem Längsschlitz oder einen geschlitzten Ring vorzusehen.

Vorzugsweise besteht der Stempelabschnitt wenigstens teilweise aus Silikon. Dies gewährleistet eine gute Dichtwirkung des Stempelabschnitts in der Injektionskartusche.

Als Hohlraumgeometrie für das Minimieren von Druckkräften des Stempelabschnitts auf die Innenwand des Kartuschenlumens der Injektionskartusche ist insbesondere eine zylinderförmiger Geometrie, eine Kegelstumpfgeometrie oder auch Blasen- oder Kugelformgeometrie günstig. So wird gewährleistet, dass der Stempelabschnitt in der Vorrichtung zum Implantieren einer Intraokularlinse mit guter Dichtwirkung durch den sich verjüngenden Bereich des Kartuschenendes bewegt werden kann, ohne dass sich der Stempelabschnitt in der Injektionskartusche verklemmt oder verkeilt.

Indem der Hohlraum in einem Frontbereich des Stempelabschnitts angeordnet ist und eine Öffnung hat, kann dieser gut mit Material, etwa Hartschaum befüllt werden.

Vorteilhafte Ausführungsformen der Erfindung sind in den Zeichnungen dargestellt und werden nachfolgend beschrieben.

Es zeigen:
Fig. 7 einen Schnitt einer Vorrichtung zum Implantieren einer Intraokularlinse mit Stempelabschnitt und Intraokularlinse in einer ersten Einstellung;
Fig. 8 einen Schnitt der Vorrichtung zum Implantieren einer Intraokularlinse mit Stempelabschnitt und Intraokularlinse in einer zweiten Einstellung;
Fig. 9 eine 3D-Schnitt Teilansicht des Stempelabschnitts bei der in Fig. 7 gezeigten Einstellung der Vorrichtung;
Fig. 10 eine 3D-Schnitt Teilansicht des Stempelabschnitts bei der in Fig. 8 gezeigten Einstellung der Vorrichtung;
Fig. 11 einen Schnitt des Stempelabschnitts in der Vorrichtung zum Implantieren einer Intraokularlinse nach Fig. 7;
Fig. 12 einen Schnitt einer zweiten alternativen Bauform eines Stempelabschnitts in einer entsprechen Fig. 7 aufgebauten Vorrichtung zum Implantieren einer Intraokularlinse;
Fig. 13 einen Schnitt einer dritten alternativen Bauform für einen entsprechenden Stempelabschnitt;
Fig. 14 und 15 eine vierte alternative Bauform für einen entsprechenden Stempelabschnitt;
Fig. 16 und 17 eine fünfte und sechste Bauform für einen Stempelabschnitt.

Die Vorrichtung 700 zum Implantieren einer Intraokularlinse in ein Auge umfasst eine röhrenförmige Injektionskartusche 702 mit einem Kartuschenlumen 703. In dem Kartuschenlumen 703 ist eine Intraokularlinse 701 angeordnet. Die Injektionskartusche 702 hat als mit Verjüngung ausgebildetes distales Kartuschenende einen Frontabschnitt 705, dessen Querschnitt 704 sich verjüngt. Am Ende des Frontabschnitts 705 hat die Injektionskartusche 702 eine Austrittsöffnung 706. Um die Intraokularlinse 704 in der Injektionskartusche 702 entsprechend dem Pfeil 715 in Richtung der Austrittsöffnung 706 zu bewegen, ist ein Stößel 707 vorgesehen. Der Stößel 707 ist aus festem, stabilem und inkompressiblem Material ausgeführt. An dem distalen Ende 709 des Stößels 707 ist ein Stempelabschnitt 710 aus Silikon angeordnet. Der Stempelabschnitt 710 hat eine zylinderförmige Bohrung 711, in welcher der Stößel 707 aufgenommen ist. In dem der Intraokularlinse 701 zugewandten Bereich des Stempelabschnitts ist ein Hohlraum 720 ausgebildet. Dieser Hohlraum 720 hat eine zylinderförmige Geometrie und ist mit Hartschaum befüllt. Mit einer Stirnseite 712 wirkt der Stempelabschnitt 710 auf die Intraokularlinse 701.

Fig. 8 zeigt die Vorrichtung 700 in der Einstellung, bei welcher der Stößel 711 in die Injektionskartusche 702 bewegt ist, um eine Intraokularlinse 701 in das Auge eines Patienten zu injizieren. Der Stempelabschnitt 710 liegt mit seiner Wandung 714 unter Dichtwirkung an der Innenwandung 715 der Injektionskartusche 702 an und verformt sich im Frontabschnitt 705 der Injektionskartusche 702. Dabei erzeugt der Stempelabschnitt 710 Druckkräfte 730 auf die Innenwandung 715 der Injektionskartusche 702. Im Vergleich zu der anhand von Fig. 1 und Fig. 2 erläuterten Vorrichtung zum Implantieren einer Intraokularlinse sind diese Druckkräfte 730 minimiert, da das Silikon des Stempelabschnitts 710 in dem mit Verjüngung ausgebildeten Frontabschnitt 705 der Injektionskartusche 702 in den Hohlraum 720 ausweichen kann, wodurch dieser deformiert wird.

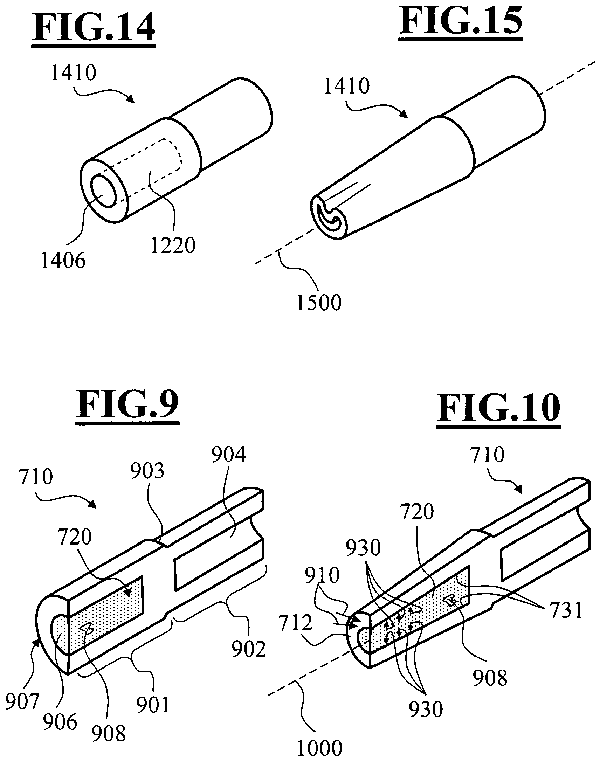

Fig. 9 zeigt eine 3D-Schnitt Teilansicht des Stempelabschnitts 710 aus Fig. 7. Der Stempelabschnitt 710 ist mit einem ersten zylindrischen Abschnitt 901 ausgebildet, der auf die Intraokularlinse wirkt, und mit einem zweiten zylindrischen Abschnitt 902, in welchem der in Fig. 7 gezeigte Stößel 707 der Vorrichtung 700 zum Implantieren einer Intraokularlinse aufgenommen ist. Hierzu ist in dem Abschnitt 902 eine Bohrung 904 vorgesehen, in welche der Stößel 707 der Vorrichtung 700 zum implantieren einer Intraokularlinse eingreift. Der Außendurchmesser des Abschnitts 901 ist größer als der Außendurchmesser des Abschnitts 902. Somit gibt es bei dem Stempelabschnitt 710 eine Stufe 903.

In dem Abschnitt 901 des Stempelabschnitts 710 befindet sich der Hohlraum 720. Der Hohlraum 720 hat eine Öffnung 906 im Frontbereich 907 des Stempelabschnitts 710.

Der Hohlraum 720 enthält eine Materialfüllung 908 aus Schaumstoff in form von Hartschaum, die als Spannelement wirkt. Durch die Öffnung 906 im Frontbereich 907 des Stempelabschnitts 710 kann der Hartschaum auf einfache Weise in den Hohlraum 720 eingebracht werden. Die Materialfüllung 908 wirkt einer Deformation von Abschnitt 901 des Stempelelements 900 entgegen.

Die Fig. 10 zeigt als 3D-Schnitt Teilansicht den Stempelabschnitt 710 aus Fig. 9 in deformiertem Zustand, wenn sich dieses, wie in Fig. 8 gezeigt, in dem sich verjüngenden Frontabschnitt 705 der Injektionskartusche 702 aus Fig. 7 befindet. Die Materialfüllung 908 erzeugt Kräfte 930, die radialsymmetrisch zur Symmetrieachse 1000 des Stempelabschnitts auf die Hohlraumwand 731 wirken. Darüber hinaus stabilisiert die Materialfüllung 908 den Stempelabschnitt 710 in Richtung der Achse 1000: Dies bewirkt, dass Kräfte 910, die auf die Stirnseite 712 des Stempelabschnitts 710 wirken, den Stempelabschnitt 710 nicht in Richtungen senkrecht zur Achse 1000 verbiegen können.

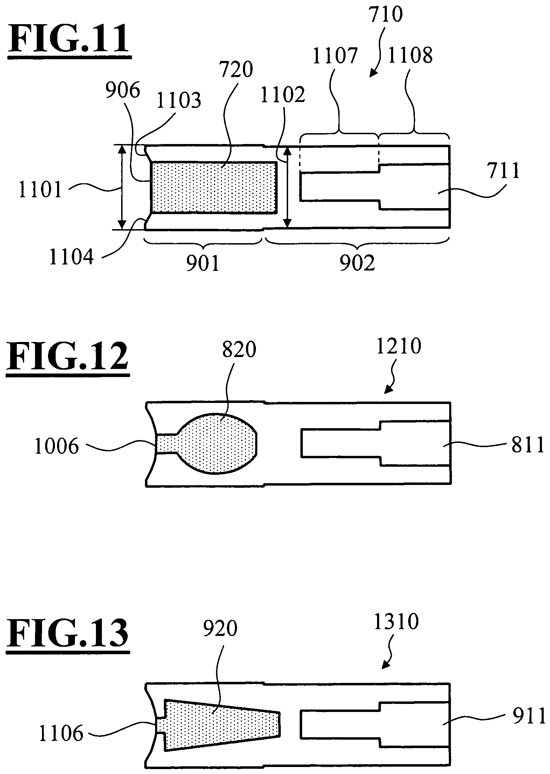

Die Fig. 11 zeigt einen Schnitt des Stempelabschnitts 710 mit zylinderförmigem Hohlraum 720 der Vorrichtung 700 zum Implantieren einer Intraokularlinse 701 aus Fig. 7.

Der Stempelabschnitt 710 hat einen Abschnitt 903 mit einem ersten Außendurchmesser 1101 sowie einen Abschnitt 902 mit einem zweiten Außendurchmesser 1102. Der Frontabschnitt des Stempelelements 907 ist mit schrägen Phasen 1103 und 1104 ausgebildet. Auf diese Weise wird ein sicheres Führen der in Fig. 7 gezeigten Intraokularlinse 701 in der Injektionskartusche 702 ermöglicht.

Der Außendurchmesser 1101 des Abschnitts 901 beträgt vorzugsweise 2.5 mm. Er ist etwas größer als der Außendurchmesser 1102 des Abschnitts 902. Eine günstige Länge für den Stempelabschnitt ist 9.5 mm.

Im Abschnitt 902 des Stempelabschnitts 710 befindet sich eine Bohrung 711 zur Aufnahme des Stößels 707 aus Fig. 7. Diese Bohrung hat einen ersten Abschnitt 1107 mit einem ersten Durchmesser und einem zweiten Abschnitt 1108 mit einem entsprechend größerem Durchmesser.

Die Öffnung des Hohlraums 720 ist mit dem Bezugszeichen 906 kenntlich gemacht.

Fig. 12 zeigt einen Schnitt einer alternativen Ausführungsform 1210 für einen als Stempelelement wirkenden Stempelabschnitt in der Vorrichtung 700 aus Fig. 7.

Soweit Abschnitte des Stempelabschnitts 1210 funktional Abschnitten im Stempelabschnitt 720 aus Fig. 11 entsprechen, sind diese mit um die Zahl 100 erhöhten Bezugszahlen kenntlich gemacht. In dem Stempelelement 1210 ist ein blasenförmiger Hohlraum 1220 ausgebildet, in dem sich wiederum ein Spannelement in Form von Hartschaum befindet.

Die Fig. 13 zeigt einen Schnitt einer weiteren alternativen Ausführungsform 1310 für ein als Stempelelement wirkenden Stempelabschnitt in der Vorrichtung 700 aus Fig. 7. Soweit Abschnitte des Stempelelements 1310 funktionalen Abschnitten des Stempelelements 720 aus Fig. 11 entsprechen sind diese mit um die Zahl 200 erhöhten Bezugszahlen kenntlich gemacht. In dem Stempelelement 1310 ist ein Hohlraum 1320 mit Kegelstumpfgeometrie ausgebildet, in dem sich als Spannelement Hartschaum befindet.

Die Fig. 14 zeigt eine weitere alternative Ausführungsform für einen Stempelabschnitt in der in Fig. 7 gezeigten Vorrichtung 700 zum Implantieren einer Okularlinse. Dieser Stempelabschnittt 1410 entspricht in seinem Aufbau dem in Fig. 9 gezeigten Stempelabschnitt 710. Soweit Abschnitte des Stempelabschnitts 1410 funktional Abschnitten im Stempelabschnitt 710 aus Fig. 9 entsprechen, sind die im Vergleich zu Fig. 9 mit um die Zahl 500 erhöhten Bezugszahlen kenntlich gemacht. Anders als der Stempelabschnitt 710 nach Fig. 9 ist beim Stempelabschnitt 1410 jedoch nicht mit Schaumstoffmaterial befüllt.

Die Fig. 15 zeigt den Stempelabschnitt 1410 im deformierten Zustand. Da bei dem Stempelabschnitt 1410 kein Spannelement vorgesehen ist, das bei Deformation des Hohlraums 1220 eine auf die Hohlraumwand gerichtete Kraft ausübt, kann sich der Stempelabschnitt bei Bewegen durch den mit Verjüngung ausgebildeten Abschnitt des Kartuschenlumens der Injektionskartusche, wie in Fig. 15 angedeutet, asymmetrisch deformieren. Es ist möglich, dass der Stempelabschnitt 1410 dabei auch im Bezug auf die Achse 1500 seitlich ausweicht. Dies beeinträchtigt einerseits die Dichtwirkung des Stempelabschnitts 1410 in der Injektionskartusche. Andererseits kann das auch zu Spitzen von Kräften auf die Innenwandung der Injektionskartusche führen, was im schlimmsten Fall bewirkt, dass diese platzt.

Bei Verwendung eines Stempelabschnitts entsprechend der Bauform nach Fig. 14 und 15 in einer Vorrichtung zum Implantieren einer Intraokularlinse in ein Auge muss daher die Wandung der Injektionskartusche vergleichsweise dick sein.

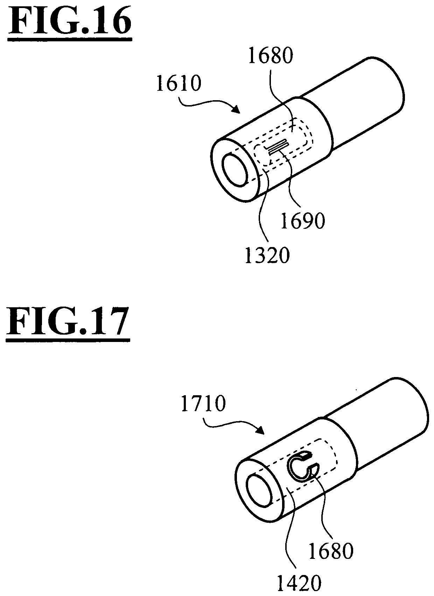

Die Fig. 16 zeigt eine weitere alternative Ausführungsform für einen Stempelabschnitt in der in Fig. 7 gezeigten Vorrichtung 700 zum Implantieren einer Okularlinse. Dieser Stempelabschnitt 1610 entspricht in seinem Aufbau dem in Fig. 9 gezeigten Stempelabschnitt 710. Soweit Abschnitte des Stempelabschnitts 1610 funktional Abschnitten im Stempelabschnitt 710 aus Fig. 9 entsprechen, sind die im Vergleich zu Fig. 9 mit um die Zahl 600 erhöhten Bezugszahlen kenntlich gemacht. In dem Stempelabschnitt 1610 ist als Spannelement eine Hülse 1680 mit Längsschlitzen 1690 vorgesehen.

Die Fig. 17 zeigt einen Stempelabschnitt 1710 für eine entsprechende Injektionskartusche mit einem geschlitzten Ring 1780 als Spannelement, der sich in einem Hohlraum 1420 befindet.

## Patentansprüche

1. Vorrichtung (700) zum Implantieren einer Intraokularlinse (701) in ein Auge
- mit einer Injektionskartusche (702); und
- mit einem durch ein Kartuschenlumen (703) schiebbaren Stößel (707), mit welchem die Intraokularlinse (701) durch ein sich verjüngendes distales Kartuschenende (705) geschoben werden kann; wobei
- an einem distalen Ende (709) des Stößels (707) ein Stempelabschnitt (710, 1210, 1310, 1410) vorgesehen ist, der unter Dichtwirkung an der Innenwand (713, 714) des Kartuschenlumens (703) anliegt und mit einer Stirnseite (712) oder einem Frontbereich (907) auf die Intraokularlinse (701) wirkt; und wobei
- in einem der Intraokularlinse zugewandten Bereich des Stempelabschnittes (710, 1210, 1310, 1410) ein Hohlraum (720, 1220, 1320, 1420) mit einer derartigen Hohlraumgeometrie ausgebildet ist, dass Druckkräfte des Stempelabschnitts (710, 1210, 1310, 1410) auf die Innenwand (713, 714) des Kartuschenlumens (703) im Bereich des sich verjüngenden Kartuschenendes (708) minimiert werden,
**dadurch gekennzeichnet, dass** der Hohlraum (720, 1220, 1320, 1420) ein Spannelement enthält, das bei einer Deformation des Hohlraums eine auf die Hohlraumwand (731) gerichtete Kraft (930) ausübt.

2. Vorrichtung nach Anspruch 1, **dadurch gekennzeichnet, dass** der Stempelabschnitt (710) eine Symmetrieachse (1000) hat und die auf die Hohlraumwand (731) gerichtete Kraft (930) bei Deformation des Stempelabschnitts (710) in dem sich verjüngenden distalen Kartuschenende im Bezug auf die Symmetrieachse (1000) radialsymmetrisch ist.

3. Vorrichtung nach Anspruch 1 oder 2, **dadurch gekennzeichnet, dass** das Spannelement eine Materialfüllung des Hohlraums (905, 1205, 1305) ist.

4. Vorrichtung nach Anspruch 3, **dadurch gekennzeichnet, dass** die Materialfüllung Hartschaum ist.

5. Vorrichtung nach Anspruch 1 oder 2, **dadurch gekennzeichnet, dass** das Spannelement eine Hülse (1680) mit wenigstens einem Längsschlitz (1690) ist oder ein geschlitzter Ring (1780) ist.

6. Vorrichtung nach einem der Ansprüche 1 bis 5, **dadurch gekennzeichnet, dass** der Stempelabschnitt (710, 1210, 1310, 1410) wenigstens teilweise aus Silikon besteht.

7. Vorrichtung nach einem der Ansprüche 1 bis 6, **dadurch gekennzeichnet, dass** der Hohlraum (720) mit zylinderförmiger Geometrie ausgebildet ist.

8. Vorrichtung nach einem der Ansprüche 1 bis 6, **dadurch gekennzeichnet, dass** der Hohlraum (1320) mit Kegelstumpfgeometrie ausgebildet ist.

9. Vorrichtung nach einem der Ansprüche 1 bis 6, **dadurch gekennzeichnet, dass** der Hohlraum (1220) blasen- oder kugelförmig ist.

## Claims

1. Device (700) for implanting an intraocular lens (701) into an eye,
- with an injection cartridge (702); and
- with a plunger (707) that can be pushed through a cartridge lumen (703), by means of which plunger the intraocular lens (701) can be pushed through a tapering distal cartridge end (705); wherein
- provision is made at a distal end (709) of the plunger (707) for a ram section (710, 1210, 1310, 1410), which rests against the inner wall (713, 714) of the cartridge lumen (703) in a sealing fashion and acts on the intraocular lens (701) with an end face (712) or a front region (907); and wherein
- a cavity (720, 1220, 1320, 1420) is formed in a region, facing towards the intraocular lens, of the ram section (710, 1210, 1310, 1410) with such a cavity geometry that pressure forces of the ram section (710, 1210, 1310, 1410) on the inner wall (713, 714) of the cartridge lumen (703) are minimized in the region of the tapering cartridge end (708),
**characterized in that** the cavity (720, 1220, 1320, 1420) contains a tensioning element, which exerts a force (930) directed at the cavity wall (731) when the cavity is deformed.

2. Device according to Claim 1, **characterized in that** the ram section (710) has an axis of symmetry (1000) and the force (930), directed at the cavity wall (731) when the ram section (710) is deformed in the tapering distal cartridge end, is radially symmetric with respect to the axis of symmetry (1000).

3. Device according to Claim 1 or 2, **characterized in that** the tensioning element is a material filling of the cavity (905, 1205, 1305).

4. Device according to Claim 3, **characterized in that** the material filling is rigid foam.

5. Device according to Claim 1 or 2, **characterized in that** the tensioning element is a sleeve (1680) with at least one longitudinal slot (1690), or a slit annulus (1780).

6. Device according to one of Claims 1 to 5, **characterized in that** the ram section (710, 1210, 1310, 1410) consists at least in part of silicone.

7. Device according to one of Claims 1 to 6, **characterized in that** the cavity (720) has a cylindrical geometry.

8. Device according to one of Claims 1 to 6, **characterized in that** the cavity (1320) has a truncated-cone geometry.

9. Device according to one of Claims 1 to 6, **characterized in that** the cavity (1220) is vesicular or spherical.

## Revendications

1. Dispositif (700) permettant d'implanter une lentille intra-oculaire (701) dans un oeil,
- comprenant une cartouche d'injection (702) ; et
- comprenant une tige poussoir (707) pouvant être poussée à travers une lumière (703) de la cartouche, avec laquelle la lentille intra-oculaire (701) peut être poussée à travers une extrémité distale rétrécie (705) de la cartouche ;
- une portion de poinçon (710, 1210, 1310, 1410) étant prévue à une extrémité distale (709) de la tige poussoir (707), laquelle s'applique avec un effet d'étanchéité contre la paroi interne (713, 714) de la lumière (703) de la cartouche et agit avec un côté frontal (712) ou une région avant (907) sur la lentille intra-oculaire (701) ; et
- une cavité (720, 1220, 1320, 1420) étant réalisée dans une région de la portion de poinçon (710, 1210, 1310, 1410) tournée vers la lentille intra-oculaire avec une géométrie de cavité telle que les forces de pression de la portion de poinçon (710, 1210, 1310, 1410) appliquées à la paroi interne (713, 714) de la lumière (703) de la cartouche dans la région de l'extrémité rétrécie (708) de la cartouche soient minimisées,
**caractérisé en ce que** la cavité (720, 1220, 1320, 1420) contient un élément de serrage qui exerce une force (930) orientée vers la paroi de la cavité (731) lors d'une déformation de la cavité.

2. Dispositif selon la revendication 1, **caractérisé en ce que** la portion de poinçon (710) présente un axe de symétrie (1000) et la force (930) orientée vers la paroi de la cavité (731), lors de la déformation de la portion de poinçon (710), présente une symétrie radiale par rapport à l'axe de symétrie (1000) dans l'extrémité distale rétrécie de la cartouche.

3. Dispositif selon la revendication 1 ou 2, **caractérisé en ce que** l'élément de serrage est un matériau de remplissage de la cavité (905, 1205, 1305).

4. Dispositif selon la revendication 3, **caractérisé en ce que** le matériau de remplissage est une mousse dure.

5. Dispositif selon la revendication 1 ou 2, **caractérisé en ce que** l'élément de serrage est une douille (1680) présentant au moins une fente longitudinale (1690) ou est une bague fendue (1780).

6. Dispositif selon l'une quelconque des revendications 1 à 5, **caractérisé en ce que** la portion de poinçon (710, 1210, 1310, 1410) se compose au moins en partie de silicone.

7. Dispositif selon l'une quelconque des revendications 1 à 6, **caractérisé en ce que** la cavité (720) est réalisée avec une géométrie de forme cylindrique.

8. Dispositif selon l'une quelconque des revendications 1 à 6, **caractérisé en ce que** la cavité (1320) est réalisée avec une géométrie de forme tronconique.

9. Dispositif selon l'une quelconque des revendications 1 à 6, **caractérisé en ce que** la cavité (1220) est réalisée en forme de bulle ou sous forme sphérique.
